# EUROPEAN PATENT APPLICATION

(11) **EP 4 492 389 A1**
(43) Date of publication of application: **15.01.2025**
(21) Application number: 23766950.2
(22) Date of filing: 09.03.2023
(51) Int. Cl.: G16C 60/00, G06F 16/23, G06N 20/00

(54) **MATERIAL CREATION SUPPORT SYSTEM, METHOD, AND PROGRAM**

(30) Priority: 10.03.2022 JP 2022037335
(71) Applicant: NGK Insulators, Ltd., Nagoya city, Aichi 467-8530 (JP)
(72) Inventor: Kimata, Takafumi, Nagoya-city, Aichi 467-8530 (JP); TOMITA, Takahiro, Nagoya-city, Aichi 467-8530 (JP); ATSUJI, Kyohei, Nagoya-city, Aichi 467-8530 (JP); MORIMOTO, Kenji, Nagoya-city, Aichi 467-8530 (JP); SOKAWA, Shingo, Nagoya-city, Aichi 467-8530 (JP); KATO, Taisuke, Nagoya-city, Aichi 467-8530 (JP); NISHIWAKI, Takafumi, Nagoya-city, Aichi 467-8530 (JP); INAGAKI, Atsunori, Nagoya-city, Aichi 467-8530 (JP)
(74) Representative: Mewburn Ellis LLP
(86) International application number: PCT/JP2023/009174
(87) International publication number: WO 2023/171774

(57) **Abstract**

A system performs either inference using a first model or inference using a second model and a third model according to accuracy of at least one model of the first to third models and, upon the inference, generates or updates recipe properties data that is data indicating an association between a manufacturing method recipe for a material and a material property/properties. The first model is a model to which a manufacturing method recipe dataset indicating a manufacturing method recipe is input and from which a material properties dataset indicating a material property/properties is output. The second model is a model to which a material characteristics dataset indicating a material characteristic(s) is input and from which a material properties dataset is output. The third model is a model to which a manufacturing method recipe dataset is input and from which a material characteristics dataset is output.

## Description

### TECHNICAL FIELD

The present invention generally relates to a technique for supporting material creation.

### BACKGROUND ART

Materials informatics (Ml) is known as one of material creation support methods. MI is a method for supporting material creation by means of an informatics technique and it is generally a method for aggregating data about material properties and searching for materials with new material properties by using machine learning (for example, PTL 1).

PTL 1: Japanese Patent Application Laid-Open (Kokai) Publication No. 2021-043959

### SUMMARY OF THE INVENTION

### PROBLEMS TO BE SOLVED BY THE INVENTION

For example, regarding organic materials, simulation of molecular properties from chemical compositions (such as first-principles calculation) is easy. Therefore, a lot of highly-accurate simulation data about properties can be acquired and it is thereby easy to apply MI.

However, the application of MI to some types of materials is difficult. For example, regarding inorganic materials (ceramics), their complicated high-order structures in which a large number of crystalline particles gather (such as their particle size, particle size distribution, and particle boundary phase) affect their properties and it is thereby difficult to simulate the properties. Therefore, it is difficult to acquire highly-accurate simulation data about the properties and it is thereby difficult to apply MI.

### MEANS TO SOLVE THE PROBLEMS

A material creation support system has first to third models and a processing unit. The first model is a model to which a manufacturing method recipe dataset indicating a manufacturing method recipe is input and from which a material properties dataset indicating a material property/properties is output. The second model is a model to which a material characteristics dataset indicating a material characteristic(s) is input and from which a material properties dataset is output. The third model is a model to which a manufacturing method recipe dataset is input and from which a material characteristics dataset is output. The processing unit performs either inference using the first model or inference using the second model and the third model according to accuracy of at least one model of the first to third models and, upon the inference, generates or updates recipe properties data that is data indicating the association between a manufacturing method recipe for a material and a material property/properties.

### ADVANTAGEOUS EFFECTS OF THE INVENTION

The present invention can contribute to a proposal of a manufacturing method recipe which supports developing a material having desired material properties.

### BRIEF DESCRIPTION OF DRAWINGS

Fig. 1 illustrates an example of a system configuration of an embodiment according to the present invention.
Fig. 2 illustrates an example of the configuration of an MI platform system 100.
Fig. 3 illustrates an example of functional blocks of an AI unit 108.
Fig. 4 illustrates an example of a flow of processing performed by a learning unit 351.
Fig. 5 illustrates an example of a flow of processing performed by an inference unit 352.
Fig. 6 schematically illustrates provision of a recipe.
Fig. 7 illustrates a variation of learning of a first model 301.

### DESCRIPTION OF EMBODIMENTS

In the description indicated below, an "interface apparatus" may be one or more interface devices. The one or more interface devices may be at least one of the following:
- One or more I/O (Input/Output) interface devices. The I/O (Input/Output) interface device is an interface device for at least one of an I/O device and a remote display computer. The I/O interface device for the display computer may be a communication interface device. At least one I/O device may be a user interface device, for example, either one of input devices such as a keyboard and a pointing device, and output devices such as a display device.
- One or more communication interface devices. The one or more communication interface devices may be one or more communication interface devices of the same type (such as one or more NICs [Network Interface Cards]) or two or more communication interface devices of different types (such as an NIC and an HBA [Host Bus Adapter]).

Furthermore, in the description indicated below, a "memory" is one or more memory devices, which are one example of one or more storage devices, and may typically be a main storage device. At least one memory device in the memory may be a volatile memory device or a nonvolatile memory device.

Furthermore, in the description indicated below, a "persistent storage apparatus" may be one or more persistent storage devices which are one example of one or more storage devices. The persistent storage device may be typically a nonvolatile storage device (such as an auxiliary storage device) and may be specifically, for example, an HDD (Hard Disk Drive), an SSD (Solid State Drive), an NVME (Non-Volatile Memory Express) drive, or an SCM (Storage Class Memory).

Furthermore, in the description indicated below, a "storage device" may be a memory and at least a memory for the persistent storage apparatus.

Furthermore, in the description indicated below, a "processor" may be one or more processor devices. At least one processor device may be typically a microprocessor device like a CPU (Central Processing Unit), but may be a processor device of a different type like a GPU (Graphics Processing Unit). At least one processor device may be of a single-core type or a multi-core type. At least one processor device may be a processor core. At least one processor device may be a processor device in a broad sense such as a circuit which is an aggregate of gate arrays by means of hardware description languages for performing a part or whole of processing (such as an FPGA [Field-Programmable Gate Array], CPLD [Complex Programmable Logic Device], or ASIC [Application Specific Integrated Circuit]).

Furthermore, in the description indicated below, a function may be sometimes described by an expression like "yyy unit"; however, the function may be implemented by execution of one or more computer programs by a processor, or may be implemented by one or more hardware circuits (such as FPGA or ASIC), or may be implemented by a combination of the above. If the function is implemented by the execution of a program by the processor, specified processing is performed by using, for example, storage devices and/or interface devices as appropriate and, therefore, the function may be considered as at least part of the processor. The processing explained by referring to the function as a subject may be the processing executed by the processor or an apparatus which has that processor. The program may be installed from a program source. The program source may be, for example, a program distribution computer or a computer-readable recording medium (such as a non-transitory recording medium). An explanation of each function is one example and a plurality of functions may be gathered as one function or one function may be divided into a plurality of functions.

An embodiment of the present invention will be described below with reference to the drawings. Incidentally, in the following description, "MI" stands for "materials informatics" and "DB" stands for "database."

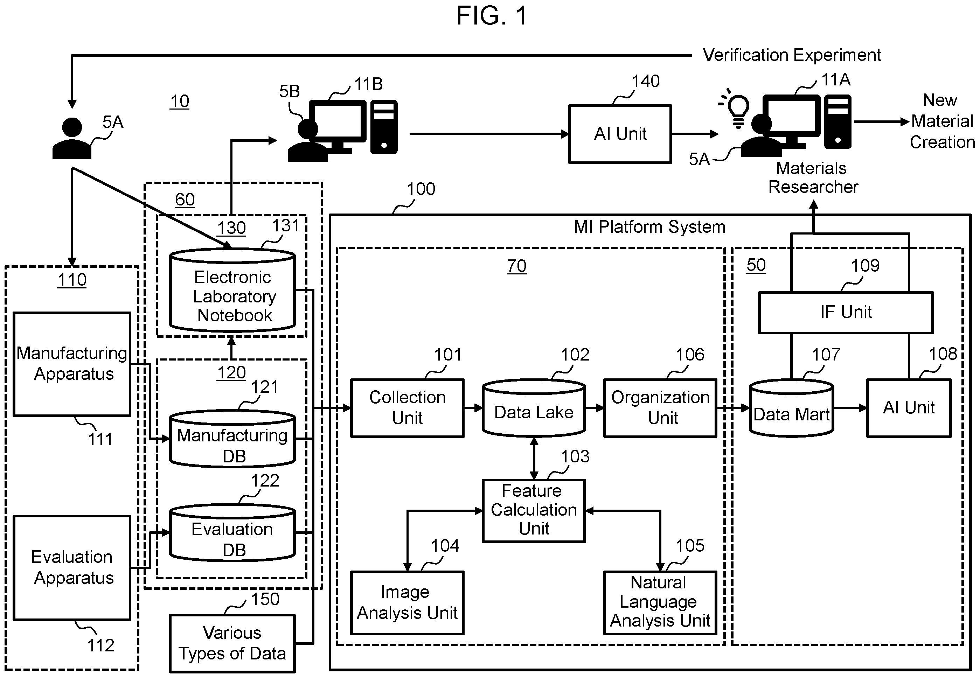

Fig. 1 illustrates an example of a system configuration of an embodiment according to the present invention.

A material creation support system 10 for supporting material creation is constructed. The material creation support system 10 may be a physical computer system, may be a logical computer system based on a physical computer system, or may be a combination of at least part of a physical computer system and at least part of a logical computer system. The physical computer system may be configured of one or a plurality of physical computers and may include an interface apparatus, a storage apparatus, and a processor connected to them. The logical computer system may include a virtual machine or may include a system as a cloud computing service.

The material creation support system 10 has a data presentation unit 50, a data acquisition unit 60, and a data conversion unit 70. These functions 50, 60, and 70 may be implemented by execution of computer programs by the processor.

The data presentation unit 50: acquires data from a data mart 107 periodically or every time it receives a query; and presents target data. Data in the data mart 107 will be hereinafter referred to as "organized data." The target data is at least either of the acquired organized data or data based on the inference result obtained by inputting the relevant organized data to a machine learning model. The data mart 107 is an example of a first data source. The relevant data should preferably include data about ceramic materials.

The data acquisition unit 60 stores experimental data which is data obtained by experiments based on the target data in an electronic laboratory notebook 131, a manufacturing DB 121, and an evaluation DB 122. The electronic laboratory notebook 131, the manufacturing DB 121, and the evaluation DB 122 are examples of a second data source. Moreover, in this embodiment, "experimental data" may be data about the experiments such as a summary, details, results, or evaluations of the experiments.

The data conversion unit 70 acquires experimental data from the electronic laboratory notebook 131, the manufacturing DB 121, and the evaluation DB 122 and converts the experimental data into data to be included in the existing or new data mart 107.

After the organized data based on the experimental data is stored in the existing data mart 107 or after a new data mart 107 including the organized data based on the experimental data is created, the target data is based on the updated or newly created data mart 107, that is, the organized data of the experimental data of the experiment(s) performed based on the target data presented in the past. Therefore, regardless of whether or not the type of material to be created is a material type which makes it possible to obtain highly-accurate simulation data about material properties, the possibility of presenting useful data for the material creation can be enhanced. As a result, material types which can support the material creation by means of the informatics technique are expanded. Specifically speaking, for example, even if the type of material to be created is an inorganic material (ceramics), data useful for the material creation can be presented.

This embodiment will be described below in detail.

Regarding the material creation support system 10, the interface apparatus may be communicably connected to at least one of the experimental system 110, the data acquisition unit 60, and the researcher terminal 11A. The storage apparatus may store at least some data of the electronic laboratory notebook 131, the manufacturing DB 121, the evaluation DB 122, various types of data 150, a data lake 102, and a data mart 107. The processor may implement the aforementioned functions 50, 60, and 70 by executing computer programs.

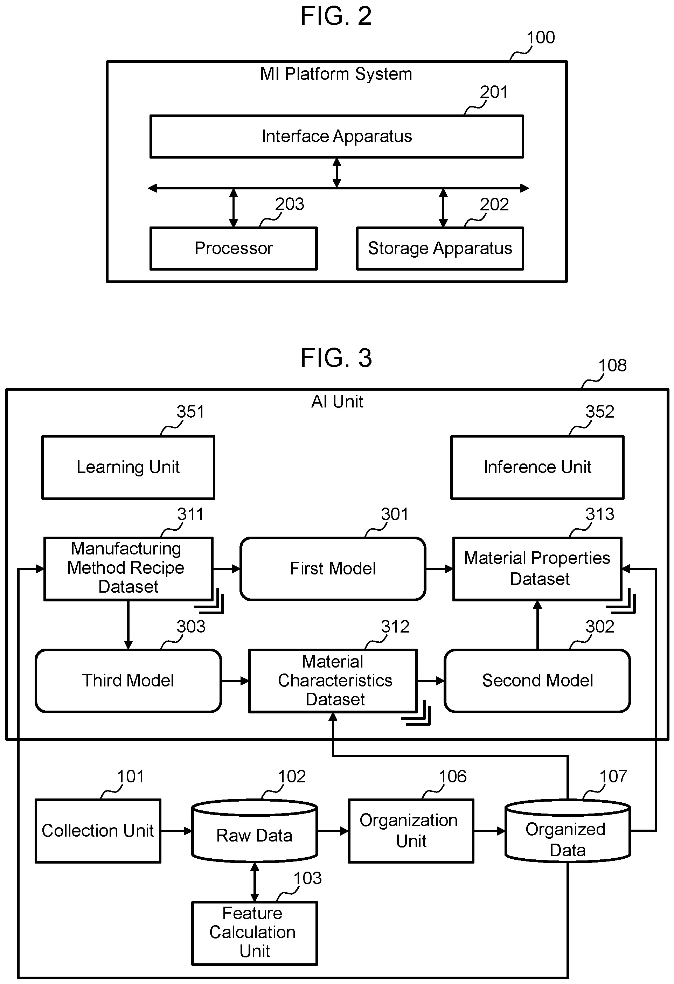

The data presentation unit 50 and the data conversion unit 70 are provided in an MI platform system 100. The data acquisition unit 60 is provided outside (or inside) the MI platform system 100. The MI platform system 100 may be a physical computer system, may be a logical computer system, or may be a combination of at least part of the physical computer system and at least part of the logical computer system. In this embodiment, as illustrated in Fig. 2, the MI platform system 100 is a physical computer system and has an interface apparatus 201, a storage apparatus 202, and a processor 203 connected to them. The MI platform system 100may communicate with a researcher terminal 11 (at least a trainee terminal 11A) through the interface apparatus 201. The storage apparatus 202 may be provided with the data lake 102 or the data mart 107 to store data. Moreover, the storage apparatus 202 may store programs. At least part of the functions of the data presentation unit 50 and the data conversion unit 70 in the MI platform system 100 may be implemented by the processor 203 by reading programs from the storage apparatus 202 and executing them.

The data presentation unit 50 has an AI (Artificial Intelligence) unit 108 and an IF (Interface) unit 109.

The AI unit 108 performs learning of a machine learning model(s) and inference using the machine learning model(s). For example, in response to an instruction from the IF unit 109, the AI unit 108 outputs to the IF unit 109 an inference result obtained by inputting the organized data acquired from the data mart 107 to the machine learning model.

The IF unit 109 receives a query from the researcher terminal 11A and presents the target data to the researcher terminal 11A in response to the query. The researcher terminal 11A is an information processing terminal (such as a personal computer or a smartphone) of a materials researcher 5A (an example of a user). The researcher terminal 11A is an example of a transmission source of a query of the target data and also an example of a presentation destination of the target data. When the IF unit 109 receives a query from the researcher terminal 11A (or periodically), it presents a target indicated by the target data (the organized data acquired from the data mart 107 and/or the data based on the inference result obtained by issuing an instruction to the AI unit 108). The presented target may be, for example, a manufacturing method recipe or a material property/properties. In this embodiment, the "manufacturing method recipe" means a material creation method and may typically include a material composition and/or a synthesis process. The "material composition" may be, for example, a compounded composition and the "synthesis process" may be raw material types (such as particle size differences), process conditions, etc. The manufacturing method recipe may include a material development strategy.

The materials researcher 5A creates a new material based on the presented target data or performs an experiment based on the target data. The presentation destination (transmission destination) of the target data may be a system like an experimental system 110 instead of or in addition to the researcher terminal 11A.

The materials researcher 5A performs an experiment using the experimental system 110 based on the presented target. For example, the experiment using the experimental system 110 may be a combinatorial experiment. The experimental system 110 may include one or a plurality of apparatuses such as a ceramic manufacturing apparatus 111 (such as an atmospheric firing furnace) and a ceramic evaluation apparatus 112 (such as an apparatus for evaluating a thermal expansion rate). Experimental data is output from the experimental apparatus(es) such as the manufacturing apparatus 111 and the evaluation apparatus 112. The output experimental data is transmitted to and stored in a data server 120. Moreover, the combinatorial experiment(s) is an example of an experiment(s) and at least one of a high-throughput experiment(s), an automatic experiment(s) using robots, and an experiment(s) mainly performed by manual work may be adopted instead of or in addition to the combinatorial experiment(s).

The data acquisition unit 60 includes the data server 120 and a laboratory notebook unit 130.

The data server 120 manages a DB in which experimental data from the experimental system 110 is stored. The DB managed by the data server 120 may exist for each experimental apparatus type. For example, the manufacturing DB 121 in which experimental data from the manufacturing apparatus 111 is stored and the evaluation DB 122 in which experimental data from the evaluation apparatus 112 is stored may be managed. The experimental data from the experimental system 110 is formed as structured data by the data server 120 and stored in the DB.

The laboratory notebook unit 130 manages experimental data acquired from a DB managed by the data server 120 as an electronic laboratory notebook 131. The electronic laboratory notebook 131 is structured data which is, for example, a DB.

The data conversion unit 70 has a collection unit 101, a feature calculation unit 103, an image analysis unit 104, a natural language analysis unit 105, and an organization unit 106.

The collection unit 101 collects the experimental data from the electronic laboratory notebook 131 and DBs of the data server 120, formats the collected experimental data, and stores the formatted experimental data in the data lake 102. The word "formatting" herein used means organizing the configuration of the experimental data into a specified configuration.

Moreover, the collection unit 101 may collect various types of data 150. The various types of data 150 may include the experimental data. Specifically speaking, for example, the various types of data 150 may include at least one of numerical value data from past experiments, materials databases available with or without charge, language data indicating past experiments (such as experiment content and experiment results), materials data obtained by calculations such as simulations, and language data from patent documents and articles. There may be one or more data sources for the various types of data 150. For example, the data sources for the numerical value data from the past experiments may be different from the data sources for the language data from patent documents and articles. The various types of data 150 may include sensor data which is data including measured values by sensors. At least part of the various types of data 150 may be stored in the data lake 102.

The feature calculation unit 103 calculates a feature(s) of one or more specified types of data in the formatted experimental data. Accordingly, each of the one or more types of data is quantified and, as a result, processing by the AI unit 108 becomes possible. For example, one or more types of data may be image data and text data. The image data is analyzed by the image analysis unit 104 and the feature calculation unit 103 calculates the feature(s) of the image data based on the analysis result. Moreover, the text data is text mined by the natural language analysis unit 105, and the feature calculation unit 103 calculates the feature(s) of the text data based on the text mining result. The aforementioned neural network may be used for calculating the feature(s). A duplicate of the experimental data may be generated in the data lake 102 and each of the one or more specified types of data in the duplicate may be converted into a calculated feature(s) (numerical value(s)).

The organization unit 106 organizes the data lake 102 into one or a plurality of data marts 107 as a set of data conforming to a specified condition(s). For example, a data mart 107 including organized data which can be presented as at least part of the target data not via the AI unit 108, and a data mart 107 including organized data which can be presented as at least part of the target data via the AI unit 108 may be generated.

Thus, the organized data based on the experimental data collected and formatted in the MI platform system 100 is prepared and the target data based on the organized data is presented to the materials researcher 5A by the MI platform system 100.

Moreover, the presentation of the data to the materials researcher 5A may also be performed not via the MI platform system 100. For example, another materials researcher 5B may acquire experimental data from the electronic laboratory notebook 131 by using a trainee terminal 11B, organize the experimental data, and input such data to the AI unit 140. The AI unit 140 may be implemented outside (or inside) the trainee terminal 11B. Similarly to the AI unit 108, the AI unit 140 may also perform inference by inputting the input organized data to a machine learning model. The data based on this inference result may be presented to the materials researcher 5A. The materials researcher 5A may perform experiments based on this presented data. This experimental data may also be stored in the data server 120 from the experimental system 110 and collected in the MI platform system 100.

Specified functions implemented inside or outside the researcher terminal 11A may facilitate experiments based on the presented target data. For example, the relevant functions may determine what experiments will be recommended to the materials researcher 5A, and may present the determined items to the materials researcher 5A. Alternatively, the target data may include data indicating what experiments will be recommended.

According to the above-described embodiment, it is expected that a cycle of presenting the target data, experimenting, collecting the experimental data, converting the experimental data into organized data, and presenting the target data based on the organized data can be performed at high efficiency and high speed. In this way, MI mainly based on the experimental data is implemented. Therefore, creation of a material of a material type for which it is difficult to acquire highly-accurate simulation data about material properties, for example, a material with a complicated high-order structure of ceramics can be supported.

Moreover, according to the above-described embodiment, various types of data inside or outside an organization (such as an enterprise) which provides the MI platform system 100 can be aggregated, structured, and managed in the MI platform system 100 and can be reflected in the data mart 107. Therefore, the possibility that the presented target data may be useful for the material creation will be enhanced.

The following expressions are possible based on the above-described explanation. The following expressions may include a supplementary explanation of the above-described explanation or an explanation of variations.

### <Expression 1>

A material creation support system including:
a data presentation unit (50) that: acquires data from a first data source periodically or
every time it receives a query; and presents target data which is at least either of the acquired data or data based on an inference result obtained by inputting the acquired data to a machine learning model;
a data acquisition unit (60) that stores experimental data, which is data obtained by an experiment based on the target data, in a second data source; and
a data conversion unit (70) that acquires the experimental data from the second data source and converts the experimental data into data to be included in the existing first data source or a new first data source.

### <Expression 2>

The material creation support system according to Expression 1,
wherein at least one of the data acquired from the first data source, the presented target data, the experimental data, and the data included in the existing first data source or a new first data source includes data about ceramic materials.

### <Expression 3>

A material creation support method including:
(A) acquiring data from a first data source periodically or every time a query is received; and presenting target data which is at least either of the acquired data or data based on an inference result obtained by inputting the acquired data to a machine learning model;
(B) storing the experimental data which is data obtained by an experiment based on the target data in a second data source; and
(C) acquiring the experimental data from the second data source and converting the experimental data into data to be included in the existing first data source or a new first data source.

### <Expression 4>

The material creation support method according to Expression 3,
wherein the machine learning model acquires updated data included in the existing first

data source or a new first data source by acquiring the experimental data and learns parameters.

Specifically speaking, for example, the details are as follows. The data in the existing data mart 107 is updated based on the experimental data acquired by (C) (such as the experimental data acquired by the data conversion unit 70) or a new data mart 107 including data based on the acquired experimental data is generated. Therefore, the existing or new data mart 107 includes update data which is data based on the acquired experimental data. The AI unit 108 may acquire the update data from the data mart 107 and learn (for example, update) the parameters of the machine learning model used by the AI unit 108.

### <Expression 5>

A material creation support method including:
collecting existing or new experimental data and converting them into data to be included in a first data source; and
acquiring the data from the first data source periodically or every time a query is received; and presenting target data which is at least either of the acquired data or data based on an inference result obtained by inputting the acquired data to a machine learning model.

For example, this material creation support method may be performed by the MI platform system 100. In other words, the material creation support system 10 may have the data presentation unit 50 and the data conversion unit 70, but may not have the data acquisition unit 60.

### <Expression 6>

The material creation support method according to any one of Expressions 3 to 5,
wherein at least one of the data acquired from the first data source, the presented target data, the experimental data, the data included in the existing first data source or a new first data source, and the data based on the inference result includes data about ceramic materials.

### <Expression 7>

A method for generating a material creation support model, wherein the method includes:
(A) acquiring data from a first data source periodically or every time a query is received; and presenting target data which is at least either of the acquired data or data based on an inference result obtained by inputting the acquired data to a machine learning model;
(B) storing experimental data, which is data obtained by an experiment based on the target data, in a second data source;
(C) acquiring the experimental data from the second data source and converting the experimental data into data to be included in the existing first data source or a new first data source; and
(D) executing learning processing of a machine learning model for outputting a recommended inference result indicating a manufacturing method recipe or a material composition by using learning data included in the first data source of (C).

### <Expression 8>

The method for generating the material creation support model according to Expression 7,
wherein at least one of the data acquired from the first data source, the presented target data, the experimental data, the data included in the existing first data source or a new first data source, the learning data, and the data indicating the recommended inference result includes data about ceramic materials.

### <Expression 9>

A program for causing a computer to execute the material creation support method stated in any one of Expressions 3 to 6.

### <Expression 10>

A program for causing a computer to execute the method for generating the material creation support model according to Expression 7 or 8.

Now, the AI unit 108 according to this embodiment will be described below in detail. In the following description, it is assumed that a target material to be created is ceramics. Moreover, in the following description, definitions of terms are as follows.
- A "dataset" is a block of logical electronic data as viewed from a program such as an application program and may be, for example, any one of records, files, key-value pairs, and tuples.
- "Researcher input information" is information which is input by the researcher 5A. The researcher input information is input to the IF unit 109. In this embodiment, the researcher input information is typically information indicating a manufacturing method recipe or material properties.
- "Researcher output information" is information which is output (provided) to the researcher 5A. The researcher output information is output from the IF unit 109. In this embodiment, the researcher output information is typically information indicating material properties or a manufacturing method recipe.
- A "manufacturing method recipe dataset" is a dataset indicating a manufacturing method recipe for a material. When the researcher input information is information indicating a manufacturing method recipe, the manufacturing method recipe dataset may be a dataset based on the relevant researcher input information.
- A "material properties dataset" is a dataset indicating material properties. When the researcher output information is information indicating material properties, the researcher output information may be information based on the material properties dataset.

Fig. 3 illustrates an example of functional blocks of the AI unit 108.

The AI unit 108 may receive a dataset from the IF unit 109 (that is, a dataset based on the researcher input information may be generated by the IF unit 109) or may receive the researcher input information from the IF unit 109 and generate a dataset based on that researcher input information. Moreover, the AI unit 108 may output a dataset to the IF unit 109 (that is, the researcher output information based on the dataset may be generated by the IF unit 109) or may output the researcher output information based on the dataset to the IF unit 109. Furthermore, the IF unit 109 may be included in the AI unit 108.

The AI unit 108 inputs a manufacturing method recipe dataset 311 and outputs (predicts) a material properties dataset 313 upon inference using a machine learning model. Furthermore, in this embodiment, when the researcher input information is information indicating a material property/properties, the AI unit 108 identifies a manufacturing method recipe dataset 311 of a manufacturing method recipe expected to create a material having the relevant material property/properties and outputs the manufacturing method recipe dataset 311 (or researcher output information based on the manufacturing method recipe dataset 311) to the IF unit 109. Specifically speaking, when the researcher 5A inputs information indicating a manufacturing method recipe to the MI platform system 100, the researcher 5A can receive provision of information indicating the predicted material properties of the material assumed to have been created by the relevant manufacturing method recipe from the MI platform system 100. Moreover, when the researcher 5A inputs information indicating material properties to the MI platform system 100, the researcher 5A can receive provision of information indicating the manufacturing method recipe of the material expected to create a material with the relevant material properties from the MI platform system 100.

The AI unit 108 has a learning unit 351 for performing learning of a machine learning model(s) and an inference unit 352 for performing inference using the learned machine learning model(s) (that is, the aforementioned inference). Moreover, there are first to third models 301 to 303 as machine learning models. The first to third models 301 to 303 are stored in the storage apparatus 202.

The first model 301 is a model to which a manufacturing method recipe dataset 311 is input and from which a material properties dataset 313 is output. Ceramics have complicated high-order structures in which a large number of crystal particles gather (such as particle size, particle size distribution, and particle boundary phase); however, material characteristics including such high-order structures affect material properties of ceramics, so that it is generally difficult to predict the material properties from a manufacturing method recipe with high the accuracy .

Therefore, in this embodiment, based on the fact that the material characteristics affect the material properties, in other words, there is a correlation between the material characteristics and the material properties, the second model 302 to which a material characteristics dataset 312 (a dataset indicating the material characteristics) is input and from which a material properties dataset 313 is output is prepared. Then, in order to make the material properties dataset 313 predictable from the manufacturing method recipe dataset 311 via an intermediate dataset that is the material characteristics dataset 312, the third model 303 to which a manufacturing method recipe dataset 311 is input and from which a material characteristics dataset 312 is output is prepared.

As described earlier, the "manufacturing method recipe" indicated by the manufacturing method recipe dataset 311 may include the material composition and/or the synthesis process and may specifically include, for example, one or a plurality of recipe items (such as a synthesis temperature) and a value of each of the one or plurality of relevant recipe items (typically a numerical value(s)).

The "material property/properties" indicated by the material properties dataset 313 means a property/properties indicated by a substance as a material (such as a structure) and may specifically include, for example, one or a plurality of property items (such as strength and a thermal expansion coefficient) and a value of each of the one or plurality of the property items (typically a numerical value(s)).

The "material characteristic(s)" indicated by the material characteristics dataset 312 means a physical and/or chemical state indicated by a substance as a material and may specifically include, for example, one or a plurality of characteristic items (such as crystal particle size and particle size distribution) and a value of each of the one or plurality of the characteristic items (typically a numerical value(s)). A feature(s) of each characteristic item may be included in the material characteristics dataset 312. For example, by using the second model 302, it is possible to predict the material properties which include a value(s) of one or a plurality of property items from the material characteristics including values of a plurality of characteristic items or to predict the material properties which include a value(s) of one or a plurality of property items from the material characteristics including a value of one characteristic item.

The material characteristics dataset 312 may be a dataset indicating a feature(s) calculated by the feature calculation unit 103 and may be stored in the data mart 107 and acquired from the data mart 107. One or a plurality of features indicated by the material characteristics dataset 312 are extracted from image data (such as SEM images) and spectral data by making use of data processing (such as processing using CNN) by the feature calculation unit 103. Incidentally, SEM stands for "Scanning Electron Microscope" and CNN stands for "Convolutional Neural Network."

Regarding each of the first to third models 301 to 303, the relevant model may be linear regression (such as Ridge regression, Lasso regression, and Elastic Net regression), logistic regression, SVM (Support Vector Machine), a decision tree model (such as a random forest, XGBoost (Xtreme Gradient Boosting), and LightGBM (Light Gradient Boosting Machine)), a neural network (such as CNN, RNN (Recurrent Neural Network), ResNet (Residual Network)), Bayesian optimization, k-NN (k-Nearest Neighbor), or a combination of any two or more of these models. Preferably, for example, regarding each of the first to third models 301 to 303, the relevant model may be a random forest, XGBoost, LightGBM, CNN, Bayesian optimization, or a combination of any two or more of these models. For example, the following is expected:
- Although the number of manufacturing method recipe datasets 311 as learning data is relatively small, Bayesian optimization enables prediction including prediction deviation, excels in finding data addition candidate points, and finds an optimum value while increasing data.
- Since a decision tree model (such as a random forest, XGBoost, and LightGBM) is a combination of simple binomial distribution problems, its computational cost is low and it has high accuracy. For example, XGBoost or LightGBM prevents overfitting and enhances robustness by creating a plurality of decision trees and weighing the target with prediction errors.
- CNN has a high degree of freedom, can deal with complex systems, and is useful when there are many features; and CNN can adjust the degree of overfitting by appropriately controlling the number of learning times (typically, the number of epochs).

In this embodiment, each of the first to third models 301 to 303 is a regression equation.

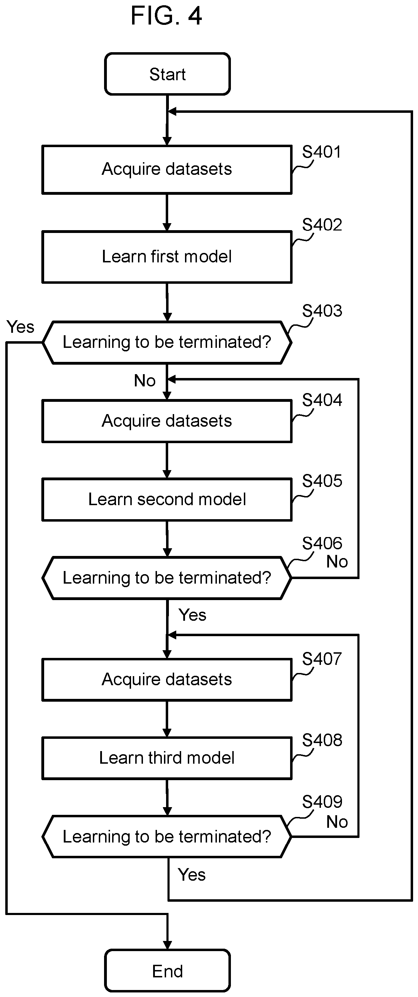

Fig. 4 illustrates an example of a flow of processing performed by the learning unit 351.

The learning unit 351 acquires learning data of the first model 301, specifically, one or more manufacturing method recipe datasets 311 and one or more material properties datasets 313 corresponding to the one or more manufacturing method recipe datasets 311 (S401). These datasets 311 and/or 313 may be input from the researcher 5A in learning processing, may be prepared in advance as learning data, may be acquired from data such as experimental data and/or various types of data 150, or may be a manufacturing method recipe dataset 311 (a manufacturing method recipe dataset 311 newly generated by the learning unit 351) indicating a manufacturing method recipe with narrowed-down recipe items or value ranges (or a manufacturing method recipe with expanded recipe items or value ranges) in a manufacturing method recipe indicated by a certain manufacturing method recipe dataset 311.

The learning unit 351 learns the first model 301 by using the acquired datasets 311 and 313 (S402). Specifically speaking, for example, the learning unit 351 learns the first model 301 by using the manufacturing method recipe dataset 311 and the material properties dataset 313 corresponding to that manufacturing method recipe dataset 311 (the manufacturing method recipe and the material properties of the material created by that manufacturing method recipe).

The learning unit 351 judges whether or not to terminate the learning of the first model 301 (S403). For example, if the accuracy (predicted accuracy) of the first model 301 is equal to or higher than a first accuracy threshold value (or if the accuracy of the first model 301 is higher than the accuracy of the second model 302 and the third model 303), the judgment result of S403 is true (S403: Yes). In this case, the processing of the learning unit 351 is terminated.

If the judgment result of S403 is false (S403: No), the learning unit 351 acquires learning data of the second model 302, specifically, one or more material characteristics datasets 312 and one or more material properties datasets 313 corresponding to the one or more material characteristics datasets 312 (S404). These datasets 312 and/or 313 may be input from the researcher 5A in the learning processing, may be prepared in advance as the learning data, may be acquired from data such as experimental data and/or various types of data 150, or may be a material characteristics dataset 312 (a material characteristics dataset 312 newly generated by the learning unit 351) indicating material characteristics with narrowed-down characteristic items or value ranges (or material characteristics with expanded characteristic items or value ranges) in material characteristics indicated by a certain material characteristics dataset 312.

The learning unit 351 learns the second model 302 by using the acquired datasets 312 and 313 (S405). Specifically speaking, for example, the learning unit 351 learns the second model 302 by using the material characteristics dataset 312 and the material properties dataset 313 corresponding to that material characteristics dataset 312 (the material characteristic(s) and the material property/properties of the material having such material characteristic(s)). The purpose of this learning is to find the material characteristic(s) having a strong correlation with the material property/properties, or in other words, to identify the material characteristic(s) corresponding to the material property/properties.

The learning unit 351 judges whether or not to terminate the learning of the second model 302 (S406). For example, if the accuracy (predicted accuracy) of the second model 302 is equal to or higher than a second accuracy threshold value, the judgment result of S406 is true (S406: Yes). If the accuracy of the second model 302 is equal to or higher than the threshold value, it means that an important characteristic item(s) and/or value(s) (feature(s)) has been identified. In other words, if no important characteristic item(s) and/or value(s) (feature(s)) has been identified, the accuracy of the second model 302 will typically not become equal to or higher than the threshold value.

If the judgment result of S406 is true, the learning unit 351 acquires learning data of the third model 303, specifically, one or more material characteristics datasets 312 identified upon the learning of the second model 302 and one or more manufacturing method recipe datasets 311 corresponding to the one or more material characteristics datasets 312 (S407). The manufacturing method recipe dataset(s) 311 corresponding to the material characteristics dataset(s) 312 may be, for example, the manufacturing method recipe dataset(s) 311 corresponding to the material properties dataset(s) 313 corresponding to the material characteristics dataset(s) 312. These datasets 312 and/or 311 may be datasets acquired from datasets used in this learning processing or may be a manufacturing method recipe dataset 311 (a manufacturing method recipe dataset 311 newly generated by the learning unit 351) indicating a manufacturing method recipe with narrowed-down recipe items or value ranges (or a manufacturing method recipe with expanded recipe items or value ranges) in a manufacturing method recipe indicated by a certain manufacturing method recipe dataset 311.

The learning unit 351 learns the third model 303 by using the acquired datasets 312 and 311 (S408). Specifically speaking, for example, the learning unit 351 learns the third model 303 by using the material characteristics dataset 312 and the manufacturing method recipe dataset 311 corresponding to that material characteristics dataset 312 (the material characteristic(s) and the manufacturing method recipe for creating a material having such material characteristic(s)).

Incidentally, the learning of the third model 303 (S407 and S408) may be performed in parallel with the learning of the second model 302 (S404 and S405). The material characteristics dataset 312 acquired in S407 may be a dataset indicating the feature(s) identified (narrowed down) upon the learning of the second model 302 or may be a material characteristics dataset 312 before such identification is performed.

The learning unit 351 judges whether or not to terminate the learning of the third model 303 (S409). If the accuracy (predicted accuracy) of the third model 303 is equal to or higher than a third accuracy threshold value, the judgment result of S409 is true (S409: Yes). Incidentally, the first to third accuracy threshold values described above may be the same value or different values.

If the judgment result of S409 is true, the learning unit 351 performs S401 again. In S401, the manufacturing method recipe dataset 311 identified (or newly generated) upon the learning of the third model 303 and the material properties dataset 313 corresponding to the above-mentioned manufacturing method recipe dataset 311 may be acquired.

According to the processing illustrated in Fig. 4, as described above, when the accuracy of the first model 301 becomes equal to or higher than the first accuracy threshold value and the learning is to be terminated, the processing of the learning unit 351 is terminated. Incidentally, when the accuracy of the first model 301 is lower than the first accuracy threshold value (if the judgment result of S403 is not true) even if the judgment of S403 is performed N times (where N is an integer equal to or more than 2), the processing of the learning unit 351 may be terminated.

In this embodiment, "relearning" means that the processing of the learning unit 351 is terminated once and the processing of the learning unit 351 is performed again. The relearning may be started at a specified timing. The specified timing may be, for example, at least one of the following:
- Regarding at least one of the models 301 to 303, the learning data has increased sufficiently from the previous learning (for example, the learning data has increased in excess of a certain quantity).
- The accuracy of at least one of the models 301 to 303 (such as the first model 301) has degraded to lower than the first accuracy threshold value.

The inference by the inference unit 352 may be performed in parallel with the processing of the learning unit 351. Moreover, the dataset obtained upon the inference by the inference unit 352 may be used for the learning of any one of the models 301 to 303. Specifically speaking, for example, at least one of the following may be adopted.
- If the judgment result of S403 is true (if the learning of the first model 301 has been terminated), the inference using the first model 301 may be performed and the manufacturing method recipe(s) may be provided as appropriate.
- If the judgment result of S406 is false (if the learning of the second model 302 has not been terminated), the inference using the second model 302 may be performed and the material characteristics may be provided as appropriate. The inference using the second model 302 may be possible, for example, if the difference between the accuracy of the second model 302 and the second accuracy threshold value is equal to or less than a specified difference.
- If the judgment result of S409 is false (if the learning of the third model 303 has not been terminated), the inference using the third model 303 may be performed and the manufacturing method recipe may be provided as appropriate. The inference using the third model 303 may be possible, for example, if the difference between the accuracy of the third model 303 and the third accuracy threshold value is equal to or less than a specified difference.
- If the judgment result of S409 is true (if the learning of the third model 303 has been terminated) and if the judgment result of S403 is false (if the learning of the first model 301 has not been terminated), the inference using the second model 302 and the third model 303 may be performed and the manufacturing method recipe(s) may be provided as appropriate.
- Experiments may be performed under a new condition(s) determined by the researcher 5A based on the manufacturing method recipe for which the inference was performed and which was provided, or based on the manufacturing method recipe estimated by the researcher 5A based on the material characteristic(s) for which the inference was performed and which was provided; and experimental data indicating a manufacturing method recipe(s), material property/properties, and material characteristic(s) related to such experiments may be accumulated. The manufacturing method recipe dataset(s) 311, the material characteristics dataset(s) 312, and the material properties dataset(s) 313 obtained from the experimental data may be used upon the learning or relearning to make the judgment result of any one of S403, S406, and S409 true. The new condition(s) may include a condition(s) based on the inference result or may include a condition(s) determined based on the knowledge of the researcher 5A and independently of the inference result.

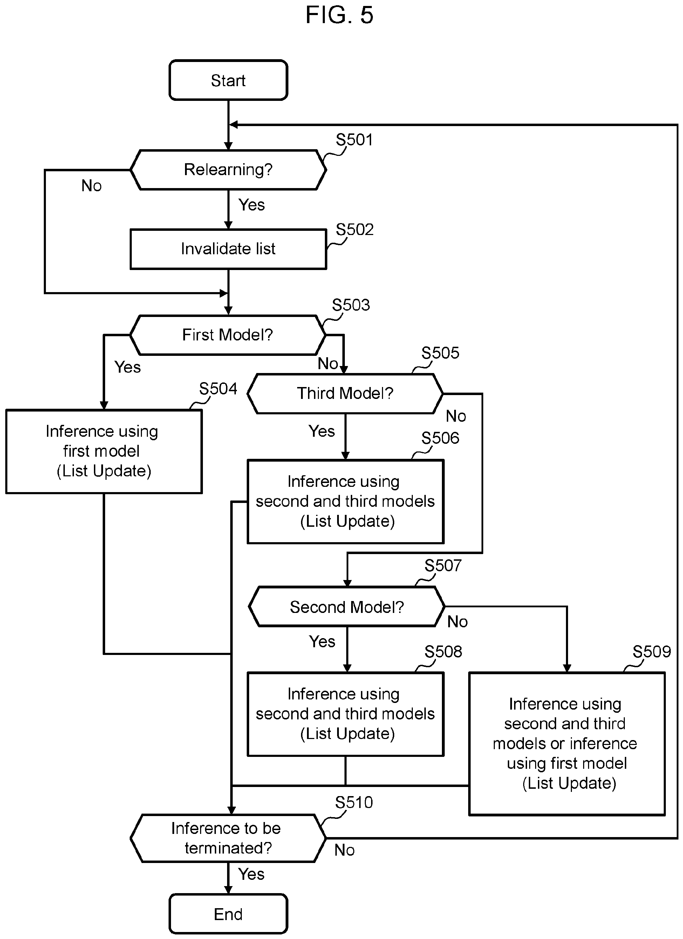

Fig. 5 illustrates an example of a flow of processing performed by the inference unit 352.

The inference unit 352 judges whether or not the relearning has been performed (S501). If the judgment result of S501 is true (S501: Yes), the inference unit 352 invalidates a list (S502). The "list" herein used is data generated or updated upon the inference and data in which the relationship between material properties and a manufacturing method recipe and/or material characteristics is recorded. The list is stored in the storage apparatus 202 by the inference unit 352 (see Fig. 2) and can be referenced by the IF unit 109. Moreover, the "invalidation" of the list means to make the list incapable of being referenced by the IF unit 109 and may specifically mean, for example, to delete the list from the storage apparatus 202, to associate the list with data indicating the invalidation, or to save the list from a certain storage area to another storage area. Furthermore, S502 may be omitted. For example, every time a list is newly generated or updated, the latest list may be validated (the list to be referenced to provide a manufacturing method recipe or material properties).

The inference unit 352 judges whether or not the learning of the first model 301 has been terminated (S503). If the judgment result of S503 is true (S503: Yes), the inference unit 352 performs the inference using the first model 301 (S504). Specifically speaking, for example, the inference unit 352 may acquire a plurality of manufacturing method recipe datasets 311 and acquire a material properties dataset 313 by inputting the manufacturing method recipe dataset 311 to the first model 301 for each acquired manufacturing method recipe dataset 311. Each of the plurality of manufacturing method recipe datasets 311 may be a dataset acquired from newly added experimental data or various types of data 150 or may be a dataset generated by changing a combination of recipe items or a value of each recipe item (for example, the inference unit 352 may increase manufacturing method recipe datasets 311 by repeatedly changing the manufacturing method recipes by, for example, increasing or decreasing the value of the synthesis temperature by a fixed value). Regarding each set of the input manufacturing method recipe dataset 311 and the acquired material properties dataset 313, the inference unit 352 records, in a list, the manufacturing method recipe indicated by the manufacturing method recipe dataset 311 and the material properties indicated by the material properties dataset 313. Moreover, the material property/properties may also be recorded in the list when the relevant material property/properties satisfy a specified condition(s) (for example, when the value of a certain property item is included within a specified value range).

If the judgment result of S503 is false (S503: No), the inference unit 352 judges whether or not the learning of the third model 303 has been terminated (S505). If the judgment result of S505 is true (S505: Yes), the inference unit 352 performs the inference using the second and third models 302 and 303 (S506). Specifically speaking, for example, the inference unit 352 acquires a material properties dataset 313 by inputting the material characteristics dataset 312 to the second model 302. In order to create a material having the material properties indicated by the acquired material properties dataset 313 (that is, in order to identify a manufacturing method recipe dataset 311 from which the material characteristics dataset 312 input to acquire the relevant material properties dataset 313 is output), the inference unit 352 may acquire the material characteristics dataset 312 by inputting the manufacturing method recipe data 311 to the third model 303. Regarding each set of the input manufacturing method recipe dataset 311, the acquired material characteristics dataset 312, and the acquired material properties dataset 313, the inference unit 352 records, in a list, the manufacturing method recipe indicated by the manufacturing method recipe dataset 311, the material characteristic(s) indicated by the material characteristics dataset 312, and the material property/properties indicated by the material properties dataset 313. In addition, the material characteristic(s) corresponding to the manufacturing method recipe and the material property/properties may also be recorded in the list. Moreover, the material property/properties may be recorded in the list when the relevant material property/properties satisfy a specified condition(s) (for example, when the value of a certain property item is within a specified value range). Furthermore, each of the plurality of manufacturing method recipe datasets 311 or material characteristics datasets 312 may be a dataset acquired from newly added experimental data or various types of data 150 or may be a dataset generated by changing a combination of respective items or a value of each item (for example, the inference unit 352 may increase manufacturing method recipe datasets 311 by repeatedly changing the manufacturing method recipes by, for example, increasing or decreasing the value of the synthesis temperature by a fixed value).

Incidentally, in S505, in addition to the judgment on whether or not the learning of the third model 303 has been terminated, the inference unit 352 may judge whether or not the accuracy of the inference using the second and third models 302 and 303 is equal to or higher than a threshold value. If this judgment result is also true, the inference unit 352 may perform S506 (the inference using the second and third models 302 and 303).

If the judgment result of S505 is false (S505: No), the inference unit 352 judges whether or not the learning of the second model 302 has been terminated (S507). If the judgment result of S507 is true (S507: Yes), the inference unit 352 performs the inference using the second model 302 and the third model 303 (S508). Specifically speaking, for example, the inference unit 352 firstly identifies a material characteristic(s) necessary to satisfy a target material property (a material characteristics dataset 312 as an input to output a material properties dataset 313 indicating the target material property) by using the second model 302. Then, the inference unit 352 uses the third model 303 to infer a manufacturing method recipe for obtaining the identified material characteristic(s) (specifically, to identify a manufacturing method recipe dataset 311 as an input to output the material characteristics dataset 312 indicating the identified material characteristic(s)). Regarding each set of the manufacturing method recipe dataset 311 as the input, the acquired material characteristics dataset 312, and the acquired material properties dataset 313, the inference unit 352 records, in a list, the manufacturing method recipe, the material characteristic(s), and the material property/properties. Incidentally, each of the plurality of manufacturing method recipe datasets 311 or the material characteristics datasets 312 may be a dataset acquired from newly added experimental data or various types of data 150 or may be a dataset generated by changing a combinations of recipe items or a value of each recipe item (for example, the inference unit 352 may increase the manufacturing method recipe datasets 311 by repeatedly changing the manufacturing method recipes by, for example, increasing or decreasing the value of the synthesis temperature by a fixed value).

If the judgment result of S507 is false (S507: No), the inference unit 352 performs the inference using two models, that is, the second and third models 302 and 303 or the first model 301 (S509). Specifically speaking, for example, the inference unit 352 performs the following (S509-1) or (S509-2). More specifically, for example, if the predicted accuracy of the second model 302 and the third model 303 is higher than the predicted accuracy of the first model 301, the inference unit 352 may perform (S509-1); and if the predicted accuracy of the second model 302 and the third model 303 is the same as or lower than the predicted accuracy of the first model 301, the inference unit 352 may perform (S509-2). Incidentally, in the following, each of the plurality of manufacturing method recipe datasets 311 or material characteristics datasets 312 may be a dataset acquired from newly added experimental data or various types of data 150 or may be a dataset generated by changing a combination of respective items or a value of each item (for example, the inference unit 352 may increase the manufacturing method recipe datasets 311 by repeatedly changing the manufacturing method recipes by, for example, increasing or decreasing the value of the synthesis temperature by a fixed value).

(S509-1) The inference unit 352 acquires a material properties dataset 313 by inputting a material characteristics dataset 312 to the second model 302. In order to create a material having the material property/properties indicated by the acquired material properties dataset 313 (in other words, in order to identify a manufacturing method recipe dataset 311 from which the material characteristics dataset 312 that is input to acquire the relevant material properties dataset 313 is to be output), the inference unit 352 acquires a material characteristics dataset 312 by inputting the manufacturing method recipe data 311 to the third model 303. Regarding each set of the input manufacturing method recipe dataset 311, the acquired material characteristics dataset 312, and the acquired material properties dataset 313, the inference unit 352 records, in a list, the manufacturing method recipe indicated by the manufacturing method recipe data 311, the material characteristic(s) indicated by the material characteristics dataset 312, and the material property/properties indicated by the material properties dataset 313.

(S509-2) The inference unit 352 acquires material properties data 313 by inputting manufacturing method recipe data 311 to the first model 301 (in actual experiments, material characteristics datasets 312 may also be collected to evaluate the material characteristics). Regarding each set of the input manufacturing method recipe dataset

311 and the acquired material properties dataset 313, the inference unit 352 records, in a list, the manufacturing method recipe indicated by the manufacturing method recipe dataset 311 and the material property/properties indicated by the material properties dataset 313.

The inference unit 352 judges whether or not to terminate the inference processing (S510). If the judgment result of S510 is true (S510: Yes), the processing is terminated. If the judgment result of S510 is false (S510: No), the processing returns to S501.

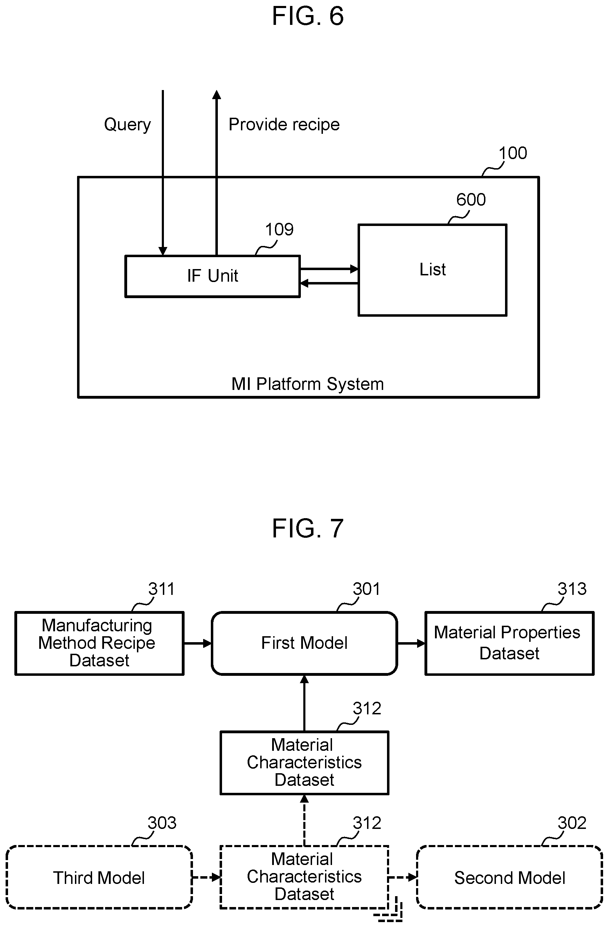

In the processing illustrated in Fig. 5, a list 600 indicated in Fig. 6 is generated or updated. When the IF unit 109 receives a query which designates a material property from the researcher 5A, it identifies a manufacturing method recipe for creating a material having the relevant material property based on the list 600 and provides the researcher 5A with the identified manufacturing method recipe.

One embodiment has been described above, but this is the illustration of an example for the purpose of explaining this invention and it is not intended to limit the scope of this invention only to this embodiment. This invention can be implemented in various other embodiments.

For example, the AI unit 140 illustrated in Fig. 1 may have the same function as the AI unit 108 described above.

Moreover, for example, the "experimental data" collected by the collection unit 101 may include at least part of data resulting from a combinatorial experiment(s), data acquired from the electronic laboratory notebook 131 in which the past experimental data are accumulated, and data acquired from, for example, an external site(s). Furthermore, the "experimental data" may include calculation data (such as data predicted by first-principles calculations or separately by machine learning) or may include language data. Furthermore, at least one of the datasets 311 to 313 may also include calculation data or language data.
- Furthermore, images may be generated upon the learning; however, in the embodiment, image generation is unnecessary upon the learning. Accordingly, the calculation load is expected to be small and the model(s) is expected to be simple.

Furthermore, for example, the aforementioned description can be summarized as follows. The following summary may include a supplementary explanation of the aforementioned description and an explanation of variations.

The material creation support system 10 may include, for example, the interface apparatus 201 connected to a user apparatus which is an apparatus having an input device and a display device (such as the researcher terminal 11A), the storage apparatus 202 in which the models 301 to 303 are stored, and the processor 203 connected to the interface apparatus 201 and the storage apparatus 202. The processor 203 may implement the AI unit 108 and the IF unit (interface unit) 109 by, for example, executing computer programs. The AI unit 108 performs either the inference using the first model 301 or the inference using the second model 302 and the third model 303 according to the accuracy (such as the predicted accuracy) of at least one model of the first to third models 301 to 303 and generates or updates the list 600 (an example of recipe properties data that is data indicating the association between a manufacturing method recipe and material properties). The IF unit 109 may receive a query which designates a material property, identify a manufacturing method recipe corresponding to the property designated by the query based on the list 600, and provide the identified manufacturing method recipe.

The second model 302 is prepared based on a correlation between material characteristics and material properties and the third model 303 is prepared to make a material properties dataset 313 predictable from a manufacturing method recipe dataset 311 via an intermediate dataset that is a material characteristics dataset 312. The presence of the second and third models 302 and 303 can contribute to a proposal of a manufacturing method recipe for creating a material with the material property/properties desired by the researcher even if the quantity of experimental data is insufficient or the accuracy of the first model 301 is insufficient due to other reasons.

If the accuracy of the first model 301 is equal to or higher than the first accuracy threshold value or the accuracy of the first model 301 is higher than the accuracy of the second and third models 302 and 303, the AI unit 108 may generate or update the list 600 upon the inference using the first model 301. Accordingly, the inference for generating or updating the list 600 used to provide a manufacturing method recipe can be performed with a smaller computational load (without input or output of the intermediate dataset as the material characteristics dataset 312). Incidentally, the "accuracy of the second model 302 and the third model 303" is not the accuracy of each individual model, that is, the accuracy of the second model 302 and the accuracy of the third model 303, but the accuracy when both the second model 302 and the third model 303 are used.

The Al unit 108 may learn the first model 301 by using the manufacturing method recipe dataset 311 and the material properties dataset 313 identified or generated upon the learning or the inference of the second and third models 302 and 303. For example, it can be specified whether it is preferable or not when, upon the learning or the inference of the second and third models 302 and 303, a recipe item that is synthesis time is present or absent in the manufacturing method recipe. Thus, upon the learning or the inference of the second and third models 302 and 303, recipe items may be expanded or reduced and the values of the recipe items may be expanded or reduced and, as a result, it is expected to increase manufacturing method recipe datasets 311 which are preferred for the learning of the first model 301. Therefore, enhancement of the accuracy of the first model 301 can be expected.

For example, since learning datasets are acquired from experimental data, insufficient experimental data can result in insufficient learning datasets and, as a result, the accuracy of the models 301 to 303 may be insufficient. Moreover, it takes time for the experimental data to become sufficient and it is difficult to guarantee when it will become sufficient. Therefore, the AI unit 108 may perform the inference using the first model 301 and/or the inference using the second and third models 302 and 303 in parallel with the learning of the models 301 to 303. For example, as the learning is switched (depending on learning of which model has been terminated), the AI unit 108 may switch (select) the model to be used for the inference.

Upon the learning of the second model 302, the AI unit 108 may identify or generate one or more material characteristics datasets 312 as a factor to cause the accuracy of the second model 302 to become equal to or higher than the second accuracy threshold value from among a plurality of material characteristics datasets 312. Upon the learning of the third model 303, the AI unit 108 may identify or generate one or more manufacturing method recipe datasets 311 corresponding to the one or more material characteristics datasets 312 identified or generated upon the learning or the inference of the second model 302 from among a plurality of manufacturing method recipe datasets 311. The AI unit 108 may learn the first model by using the one or more manufacturing method recipe datasets 311 identified or generated upon the learning or the inference of the third model 303 and one or more material properties datasets 313 corresponding to the one or more manufacturing method recipe datasets 311. Accordingly, the material characteristics datasets 312 are narrowed down upon the learning or the inference of the third model 303, so that the accuracy of the third model 303 can be enhanced with a small computational load. Moreover, the manufacturing method recipe datasets 311 corresponding to such narrowed-down material characteristics datasets 312 are used to learn the first model 301, so that the accuracy of the first model 301 can be enhanced with a small computational load.

A material characteristics dataset 312 may be a dataset configured of features as numerical values of each characteristic item belonging to the material characteristics. Accordingly, the computational load on the learning and the inference can be reduced and the second and third models 302 and 303 can be simplified.

When relearning is performed, the AI unit 108 may discard the list 600 and newly generate a list 600 or update the list 600 upon the inference using the first model 301 after the relearning and/or the inference using the second model 302 after the relearning and the third model 303 after the relearning. Accordingly, it is possible to prevent the list generated or updated upon the inference using an old model whose the accuracy was not yet improved from being provided as a recipe by the IF unit 109.

Moreover, the following variation may be used as one variation. Specifically speaking, for example, as illustrated in Fig. 7, if the accuracy of the first model 301 is lower than the first accuracy threshold value (for example, when the accuracy of the first model 301 does not become equal to or higher than the first accuracy threshold value even if the learning of the first model 301 is performed a specified number of times), in addition to the manufacturing method recipe dataset(s) 311 identified or generated upon the learning of the second and third models 302 and 303, the AI unit 108 may perform the learning of the first model 301 by using the material characteristics dataset(s) 312 corresponding to the above-mentioned manufacturing method recipe dataset(s) 311 and identified or generated upon the learning or the inference of the second model 302 as an input to the first model 301 (learning of the correspondence relationship between the set of a manufacturing method recipe and material characteristic(s), and the material property/properties). Accordingly, enhancement of the possibility of making the accuracy of the first model 301 equal to or higher than the first accuracy threshold value can be expected. Incidentally, in this case, upon the inference using the first model 301, the AI unit 108 may generate or update the list 600 in which a material characteristic(s) in addition to the manufacturing method recipe is associated with a material property/properties (in other words, the list 600 in which the relationship between the manufacturing method recipe, the material characteristic(s), and the material property/properties is recorded). The IF unit 109 may receive a query which designates a material characteristic(s) in addition to the material property/properties, identify the manufacturing method recipe corresponding to the material property/properties and material characteristic(s) designated by the relevant query based on the list 600, and provide the identified manufacturing method recipe.

For example, there may be the following use case as one use case. Specifically speaking, the material characteristics may include (1) density and porosity quantified by the Archimedean method and (2) SEM image features (such as a composition area ratio and a composition area deviation). Characteristic items (characteristic factors) which may affect objective properties (material properties desired by the researcher) may be porosity, a crystal perimeter, a composition area ratio, and a composition area deviation (the "composition area deviation" is a feature indicating variations of the composition ratio of a plurality of segmented areas constituting one image (in this case, an SEM image)). The first model 301 may be Ridge regression (its inputs may be manufacturing conditions such as a composition ratio, material relay, and a firing temperature, and its outputs may be bending strength and a thermal expansion coefficient). The second model 302 may be Ridge regression or LightGBM (its inputs may be the aforementioned (1) density and porosity and (2) SEM image features, and its outputs may be bending strength and a thermal expansion coefficient). The third model 303 may be Ridge regression (its inputs may be manufacturing conditions such as a composition ratio, a material particle size, and a firing temperature, and its outputs may be the aforementioned (1) density and porosity and (2) SEM image features). In response to a query from the researcher 5A (a query which designates a material property), a manufacturing method recipe may be provided (may be displayed on the researcher terminal 11A). Moreover, any one of the models 301 to 303 may include a regression equation for each element as an output (such as an objective variable). Specifically speaking, any one of the models 301 to 303 may include one or more regression equations. Furthermore, although any of the models 301 to 303 may have a common dataset as an input, explanatory variables and weights are typically different for the respective regression equations for the models.

Moreover, the significance of the processing illustrated in Fig. 5 is, for example, as follows.

Specifically speaking, the purposes of the inference can be (1) to find a manufacturing method recipe for manufacturing a material having a desired material property/properties and (2) to find a manufacturing method recipe candidate(s) for adding experimental data in order to enhance the model accuracy . The inference of S504 in Fig. 5 corresponds to the purpose (1) (because the model learning has been completed). On the other hand, the inferences of S506, S508, and S509 in Fig. 5 correspond to the purpose (2) (because the model learning has not been completed).

A dataset useful or effective for model learning (such as a manufacturing method recipe) is selected from among the combinations identified upon the inference corresponding to the purpose (2) (such as a combination of a manufacturing method recipe and a material property/properties), a dataset obtained by actual experiments based on the aforementioned dataset is added as a learning dataset, and model learning is performed by using the added dataset. Since the dataset is augmented in this way, the model can be learned with better the accuracy.

In the case of Yes in S505, the accuracy of the inference using the second model 302 and the third model 303 can be regarded as sufficient. Therefore, an additional learning dataset can be prepared based on the dataset input and output upon the inference. In addition to the dataset input and output upon the inference, a dataset considered and generated by a researcher (such as an experimenter) may be used to prepare an additional learning dataset.

On the other hand, in the case of No in S505, the accuracy of the inference using the second model 302 and the third model 303 can be regarded as insufficient. Therefore, it is difficult to prepare the additional learning dataset based only on the dataset input and output upon the inference and the dataset considered and generated by the researcher may be also used to prepare the additional learning dataset.

Since the learning of the second model 302 is performed before the learning of the third model 303, it is more efficient to perform the judgment of S505 before the judgment of S507. Incidentally, the order of judgments may not be limited to the sequential order indicated in Fig. 5 (that is, the sequential order of S503, S505, and then S507).

The following expressions are possible based on the above description.

If the accuracy of the first model 301 is lower than the first threshold value or the accuracy of the first model 301 is lower than the accuracy of the second model 302 and the third model 303, the AI unit 108 may perform the inference using the second model 302 and the third model 302.

The AI unit 108 may learn the second model 302 before the third model 303. The AI unit 108 may prioritize using the dataset input or output upon the inference using the second model 302 and the third model 303 to learn at least one of the first to third models regarding a second case rather than a first case.

The first case is the case where the inference using the second model 302 and the third model 303 is the inference performed when the accuracy of the third model 303 is lower than the third threshold value.

The second case is the case where the inference using the second model 302 and the third model 303 is the inference performed when the accuracy of the third model 303 is equal to or higher than the third threshold value.

When the accuracy of the second model 302 is lower than the second threshold value: and if the accuracy of the second model 302 and the third model 303 is higher than the accuracy of the first model 301, the AI unit 108 may perform the inference using the second model 302 and the third model 303; and if the accuracy of the second model 302 and the third model 303 is equal to or lower than the accuracy of the first model 301, the AI unit 108 may perform the inference using the first model 301.

### REFERENCE SIGNS LIST

- 10:: material creation support system
- 100:: MI platform system

## Claims

1. A material creation support system comprising:
a first model that is a model to which a manufacturing method recipe dataset indicating a manufacturing method recipe is input and from which a material properties dataset indicating a material property is output;
a second model that is a model to which a material characteristics dataset indicating a material characteristic is input and from which a material properties dataset is output;
a third model that is a model to which a manufacturing method recipe dataset is input and from which a material characteristics dataset is output; and
a processing unit that performs either inference using the first model or inference using the second model and the third model according to accuracy of at least one model of the first to third models and, upon the inference, generates or updates recipe properties data that is data indicating an association between a manufacturing method recipe for a material and a material property.

2. The material creation support system according to claim 1,
wherein, if the accuracy of the first model is equal to or higher than a first threshold value or the accuracy of the first model is higher than the accuracy of the second model and the third model, the processing unit performs the inference using the first model.

3. The material creation support system according to claim 2,
wherein the processing unit learns the first model by using a manufacturing method recipe dataset and a material properties dataset identified or generated upon learning or the inference of the second model and the third model.

4. The material properties support system according to claim 3,
wherein the processing unit:
upon the learning of the second model, identifies or generates one or more material characteristics datasets as a factor causing the accuracy of the second model to be equal to or higher than a threshold value, from among a plurality of material characteristics datasets;
upon the learning of the third model, identifies or generates one or more manufacturing method recipe datasets corresponding to the one or more material characteristics datasets identified or generated upon the learning or the inference of the second model,
from among a plurality of manufacturing method recipe datasets; and
learns the first model by using the one or more manufacturing method recipe datasets identified or generated upon the learning or the inference of the third model and the one or more material properties datasets corresponding to the one or more manufacturing method recipe datasets.

5. The material creation support system according to claim 1,
wherein a material characteristics dataset is a dataset configured of features as numerical values of respective characteristic items belonging to material characteristics.

6. The material creation support system according to claim 1,
wherein when performing relearning, the processing unit newly generates or updates recipe properties data upon the inference using the first model after the relearning and/or upon the inference using the second model after the relearning and the third model after the relearning.

7. The material properties support system according to claim 3,
wherein if the accuracy of the first model is lower than the threshold value, the processing unit performs learning of the first model by using, besides the manufacturing method recipe dataset identified or generated upon the learning or the inference of the second model and the third model, a material characteristics dataset which corresponds to the manufacturing method recipe dataset and is identified or generated upon the learning or the inference of the second model as an input to the first model.

8. The material properties support system according to claim 7,
wherein if the material characteristics dataset is used for learning of the first model, the processing unit generates or updates recipe properties data which associates a material characteristic, besides the manufacturing method recipe, with a material property upon the inference using the first model,.

9. The material properties support system according to claim 3,
wherein the processing unit performs the inference using the first model and/or the inference using the second model and the third model in parallel with the learning of the first model, the second model, and the third model.

10. The material properties support system according to claim 1,
wherein the processing unit receives a query which designates a material property,
identifies a manufacturing method recipe corresponding to the property designated by the query based on the recipe properties data, and provides the identified manufacturing method recipe.

11. The material properties support system according to claim 10,
wherein the processing unit:
receives a query which designates a manufacturing method recipe;
identifies a material property corresponding to the manufacturing method recipe designated by the query on the basis of the recipe properties data or identifies the material property by causing the processing unit to perform inference by inputting a manufacturing method recipe dataset indicating the manufacturing method recipe; and
provides the identified material property.

12. The material creation support system according to claim 1,
wherein if the accuracy of the first model is lower than a first threshold value or the accuracy of the first model is lower than the accuracy of the second model and the third model, the processing unit performs the inference using the second model and the third model.

13. The material creation support system according to claim 1,
wherein the processing unit learns the second model before the third model.

14. The material creation support system according to claim 13,
wherein the processing unit prioritizes using a dataset obtained by an experiment based on a dataset input or output upon the inference using the second model and the third model to learn at least one of the first to third models regarding a second case rather than a first case;
wherein the first case is a case where the inference using the second model and the third model is inference performed when the accuracy of the third model is lower than a third threshold value; and
wherein the second case is a case where the inference using the second model and
the third model is inference performed when the accuracy of the third model is equal to or higher than the third threshold value.

15. The material creation support system according to claim 14,
wherein when the accuracy of the second model is lower than a second threshold value: and
if the accuracy of the second model and the third model is higher than the accuracy of the first model, the processing unit performs the inference using the second model and
the third model; and
if the accuracy of the second model and the third model is equal to or lower than the accuracy of the first model, the processing unit performs the inference using the first model.

16. A material creation support method comprising:
causing a computer to perform either inference using a first model or inference using a second model and a third model according to accuracy of at least one model of the first to third models;
upon the inference, causing the computer to generate or update recipe properties data that is data indicating an association between a manufacturing method recipe for a material and a material property;
wherein the first model is a model to which a manufacturing method recipe dataset indicating a manufacturing method recipe is input and from which a material properties dataset indicating a material property is output;
wherein the second model is a model to which a material characteristics dataset indicating a material characteristic is input and from which a material properties dataset is output; and
wherein the third model is a model to which a manufacturing method recipe dataset is input and from which a material characteristics dataset is output.

17. A computer program for causing a computer to:
perform either inference using a first model or inference using a second model and a third model according to accuracy of at least one model of the first to third models; and upon the inference, generate or update recipe properties data that is data indicating an association between a manufacturing method recipe for a material and a material property,
wherein the first model is a model to which a manufacturing method recipe dataset indicating a manufacturing method recipe is input and from which a material properties dataset indicating a material property is output;
wherein the second model is a model to which a material characteristics dataset indicating a material characteristic is input and from which a material properties dataset is output; and
wherein the third model is a model to which a manufacturing method recipe dataset is input and from which a material characteristics dataset is output.

18. A storage medium with a computer program stored therein for causing a computer to:
perform either inference using a first model or inference using a second model and a third model according to accuracy of at least one model of the first to third models; and
upon the inference, generate or update recipe properties data that is data indicating an association between a manufacturing method recipe for a material and a material property,
wherein the first model is a model to which a manufacturing method recipe dataset indicating a manufacturing method recipe is input and from which a material properties dataset indicating a material property is output;
wherein the second model is a model to which a material characteristics dataset indicating a material characteristic is input and from which a material properties dataset is output; and
wherein the third model is a model to which a manufacturing method recipe dataset is input and from which a material characteristics dataset is output.
